# FASCICULE DE BREVET EUROPEEN

(11) **EP 1 090 651 B1**
(45) Date de publication et mention de la délivrance du brevet: **09.06.2004**
(21) Numéro de dépôt: 00402594.6
(22) Date de dépôt: 20.09.2000
(51) Int. Cl.: A61M 5/30, F42B 3/117

(54) **Seringue sans aiguille munie d'un système de déclenchement par friction.**
Nadellose Spritze mit Reibungstriggerung
Needleless syringe supplied with a trigger activated by friction

(30) Priorité: 08.10.1999 FR 9912559
(43) Date de publication de la demande: 11.04.2001
(73) Titulaire: Crossject, 75181 Paris Cédex 04 (FR)
(72) Inventeur: Alexandre, Patrick, 70100 Gray (FR); Brouquieres, Bernard, 83100 Toulon (FR); Gautier, Philippe, 91120 Le Plessis Pate (FR)
(74) Mandataire: Pech, Bernard

(56) Documents cités:
- CH-A- 681 175
- FR-A- 2 487 968
- US-A- 3 145 712
- US-A- 3 308 818
- US-A- 3 335 722
- US-A- 4 089 334

## Description

Le domaine technique de l'invention est celui des seringues sans aiguille destinées à injecter, à travers la peau, des particules solides, liquides ou mixtes de principe actif à usage thérapeutique.

En effet, l'invention se rapporte à une seringue sans aiguille fonctionnant à partir d'un dispositif d'initiation faisant intervenir un dispositif de déclenchement associé à une charge pyrotechnique. Plus spécialement, ce dispositif de déclenchement doit être adapté à la mise à feu d'une charge pyrotechnique logée dans un objet léger, de petite taille et devant être actionné manuellement.

La solution proposée par l'invention prône l'utilisation d'un rugueux comme pièce principale du dispositif de déclenchement de la seringue sans aiguille.

Il s'avère que dans le domaine des seringues sans aiguille, aucun brevet ne se rapporte à la mise en oeuvre d'un rugueux dans le dispositif de déclenchement de telles seringues. Cependant, d'autres types de dispositif de déclenchement ont déjà été mis au point pour des seringues sans aiguille faisant intervenir une charge pyrotechnique. On peut citer, par exemple, le brevet US 2 322 244 relatif à un injecteur hypodermique sans aiguille fonctionnant à partir d'une cartouche à blanc, dont la mise à feu est provoquée par la percussion d'un piston mis en vitesse par la détente d'un ressort, celle-ci étant déclenchée manuellement par contraction préalable dudit ressort à l'aide d'un bouton. Le liquide à injecter étant placé au contact de la cartouche, est expulsé de l'injecteur sous l'effet de la pression générée par les gaz de combustion. Le brevet US 4 089 334, quant à lui, décrit un injecteur sans aiguille munie d'une charge pyrotechnique dont la mise à feu est assurée par une charge primaire, elle-même initiée par percussion d'une tige rigide mise en mouvement par la détende d'un ressort. Les gaz émis par la charge pyrotechnique s'expansent dans une chambre aval, mettant en mouvement un piston dont le déplacement provoque l'expulsion du produit liquide à injecter. Ces deux brevets tendent à montrer que les dispositifs de déclenchement par percussion faisant notamment intervenir un ressort, une pièce solide servant de percuteur et au moins une charge pyrotechnique, sont bien connus dans le domaine des seringues sans aiguille.

Enfin, il est intéressant de mentionner le brevet US 3 145 712 qui se rapporte à un dispositif d'injection de médicament transcutanée destiné à compléter les tenues de protection aux agressions gazeuses. Ces dispositifs sont portés par l'individu et peuvent être déclenchés soit manuellement, soit automatiquement en cas de détection gazeuse. Ces dispositifs fonctionnent à partir d'une cartouche pyrotechnique mise à feu par une charge primaire, celle-ci étant initiée électriquement par un courant créé entre deux bornes judicieusement placées au sein de cette charge primaire. Les gaz générés par la combustion de la charge pyrotechnique créés une pression servant à éjecter le produit médicamenteux liquide.

En revanche, l'utilisation d'un rugueux pour initier une charge pyrotechnique dans des objets tels que, par exemple, des fusées éclairantes, des grenades ou des mines est connue, et a fait l'objet de plusieurs brevets. On peut citer, entre autres, le brevet français FR 2 393 261 qui se rapporte à une mine indétectable pour chenille pouvant être déclenchée par l'intermédiaire d'un frottoir dont le déplacement au contact d'une amorce va contribuer à son initiation.

De même, le brevet français FR 2 487 968 décrit un bouchon allumeur particulièrement adapté à la mise à feu de grenades et contenant notamment une chaîne pyrotechnique d'allumage dont le premier élément est une charge pyrotechnique pouvant être initiée par friction.

Les seringues sans aiguille doivent être dotées d'un dispositif de déclenchement pouvant être actionné manuellement et permettant de s'affranchir d'une source d'activation trop énergétique ou trop encombrante tout en restant fiable et performant. En effet, les dispositifs de déclenchement habituellement utilisés font intervenir des amorces dont la composition primaire est particulièrement sensible. En situation de stockage, ces seringues peuvent alors présenter un risque de fonctionnement intempestif, en raison de la grande sensibilité de l'amorce à des sollicitations extérieures telles que des températures élevées ou des chutes. D'autre part, malgré leur sensibilité, les amorces nécessitent, pour être initiées, un dispositif de percussion relativement encombrant. De façon à résoudre ce problème d'encombrement et ce risque de fonctionnement intempestif, il a été découvert la possibilité de miniaturiser une initiation par friction, réalisée avec une bonne sécurité, et se déclenchant avec une excellente fiabilité. Cette initiation par friction s'effectue en douceur, par inflammation directe de la charge pyrotechnique, sans avoir recours à une onde de choc comme dans le cas d'une initiation avec une amorce. Ceci a pour conséquence directe et avantageuse, un effet sonore extrêmement limité lors de l'utilisation de la seringue. Enfin, la seringue sans aiguille selon l'invention possédant un dispositif de déclenchement par friction, présente une double sécurité, l'une, sous la forme d'une pièce escamotable empêchant tout enfoncement du déclencheur, l'autre, sous la forme d'une zone de rugosité nécessitant un effort particulier pour enfoncer le déclencheur une fois que la pièce escamotable a été retirée.

La seringue sans aiguille selon l'invention possède toutes ces caractéristiques techniques particulièrement adaptées à l'injection sans aiguille.

L'objet de la présente invention concerne une seringue sans aiguille munie d'un dispositif d'initiation comprenant un dispositif de déclenchement et une charge pyrotechnique caractérisée en ce que le dispositif de déclenchement comprend un organe de déclenchement relié à un moyen de friction de la charge pyrotechnique. Le moyen de friction est constitué par une pièce dont la fonction principale est de venir se frotter contre une charge pyrotechnique dans le but de provoquer son initiation directe. Un tel dispositif requiert une composition pyrotechnique sensible à la friction comme par exemple des compositions à base de Zirconium. Avantageusement, le moyen de friction est un rugueux. De façon préférentielle, le rugueux est constitué par une pièce allongée dont une extrémité présente des aspérités. Préférentiellement, la pièce est rigide et a une forme sensiblement cylindrique. De façon avantageuse, la pièce est solidaire de l'organe de déclenchement de sorte que son déplacement est dicté par le mouvement imprimé au dit organe.

Selon un mode de réalisation préféré de l'invention, l'organe de déclenchement est un bouton poussoir destiné à être actionné manuellement et pouvant coulisser le long d'un corps central creux allongé dans lequel est logée la charge pyrotechnique. En configuration « stockage» pour laquelle la seringue sans aiguille n'a pas encore fonctionné, le rugueux est distant de la charge pyrotechnique. L'allumage de celle-ci va se produire lorsque le rugueux arrive à son contact en provoquant un frottement mécanique direct.

Préférentiellement, le bouton poussoir est placé à l'une des extrémités du corps central pour faciliter son actionnement, et plus spécialement à l'extrémité opposée à celle par laquelle le principe actif est injecté. Avantageusement, le rugueux et la charge pyrotechnique sont alignés suivant l'axe du corps creux. De façon préférentielle, le rugueux et la charge pyrotechnique ont une forme sensiblement cylindrique et leur axe est confondu avec l'axe du corps creux. De façon avantageuse, une zone de rugosité située entre le bouton poussoir et le corps central permet d'accroître les forces de frottement entre ces deux éléments, en cas de coulissement de l'un sur l'autre. De façon plus concrète, cette zone permet d'introduire une résistance, empêchant toute initiation tant qu'un effort suffisant n'est pas appliqué suffisamment longtemps pour enfoncer le rugueux au contact de la charge pyrotechnique.

De façon préférentielle, la zone de rugosité est constituée par l'emboîtement mutuel d'aspérités annulaires de l'un des deux éléments dans des rainures de l'autre élément prévues pour les recevoir, de façon à induire un niveau de pression minimal pour commencer à enfoncer le bouton poussoir. Cela évite un déclenchement intempestif à partir d'une sollicitation anodine ou involontaire lorsque la seringue sans aiguille est rendue opérationnelle par le retrait de la butée escamotable qui protège l'extrémité par laquelle le principe actif est injecté.

En effet, préférentiellement, le bouton poussoir dispose d'une sûreté sous la forme d'une butée escamotable empêchant tout mouvement dudit bouton. L'autre fonction de cette sûreté est de protéger l'extrémité de la seringue par laquelle doit s'éjecter le principe actif.

Avantageusement, la butée escamotable est constituée par un bouchon muni d'une collerette bloquant lé bouton poussoir. Une ligne de faiblesse circulaire permet de dissocier la collerette du bouchon. La collerette est rigide et comporte une tirette pour la déchirer. De façon avantageuse, les aspérités annulaires constituant la zone de rugosité, ont la particularité de se déformer préférentiellement suivant l'axe de poussée et d'avoir, par conséquent, un effet anti-retour. De cette manière, l'utilisateur ne risque pas de subir un effet brutal de recul du bouton poussoir. De façon préférentielle, le rugueux est en acier inoxydable.

Les seringues sans aiguille selon l'invention présentent l'avantage de produire une réaction pyrotechnique moins violente que celle qui est perçue lors du fonctionnement d'une amorce à percussion. En effet, elles permettent une initiation en douceur de la composition génératrice de gaz par l'intermédiaire d'un frottement et non plus par l'intermédiaire d'une onde de choc.

De plus, lors de leur déclenchement et de leur fonctionnement, elles engendrent un effet sonore extrêmement limité.

Enfin, elles présentent l'avantage d'être mises sur le marché civil sans subir les contraintes inhérentes aux dispositifs impliquant des explosifs ou des compositions détonantes.

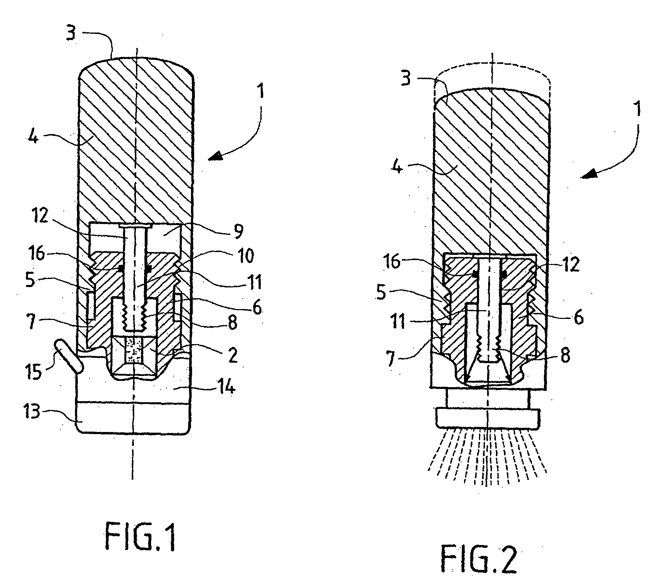

On donne ci-après, la description détaillée d'un mode de réalisation préférée de l'invention en se référant aux figures 1 et 2.

La figure 1 est une vue en coupe axiale longitudinale d'une seringue sans aiguille selon l'invention n'ayant pas encore fonctionné.

La figure 2 est une vue en coupe axiale longitudinale de la seringue de la figure 1 ayant fonctionné.

En se référant à la figure 1, selon le mode de réalisation préférée de l'invention, la seringue sans aiguille 1 présente une partie amont comprenant un dispositif de déclenchement, et une partie aval comprenant une charge pyrotechnique 2, le principe actif sous forme solide ou liquide, une buse d'éjection et un guide d'application sur la peau. Le dispositif de déclenchement inclut un bouton poussoir 3 et un moyen de friction sous la forme d'un rugueux 12.

Le bouton poussoir 3 a une forme sensiblement cylindrique et est constitué par une partie pleine 4 prolongée par une partie cylindrique creuse 5 de diamètre extérieur identique. Cette partie cylindrique creuse 5 de diamètre extérieur constant comporte un épaulement interne permettant de distinguer un cylindre arrière creux de forte épaisseur en continuité avec un cylindre avant creux de plus faible épaisseur, le cylindre arrière étant compris entre la partie pleine 4 et le cylindre avant. Le cylindre arrière possède sur sa paroi latérale interne une zone présentant une série d'aspérités annulaires. La partie cylindrique creuse 5 enserre, sur une partie de sa longueur, un corps cylindrique creux 6 possédant sur sa paroi latérale externe une zone présentant également une série d'aspérités annulaires, ledit corps 6 étant prolongé par une embase cylindrique élargie 7.

Le corps cylindrique creux 6 comporte un épaulement interne permettant de distinguer un canal amont de faible diamètre en continuité avec un canal aval de plus fort diamètre dans lequel est logée la charge pyrotechnique 2. Cette charge pyrotechnique 2 se divise en deux parties distinctes, l'une, relative à une composition à base de Zirconium sensible à la friction, et l'autre, à une composition génératrice de gaz qui servira à expulser les particules. Le rugueux 12 est constitué par une pièce allongée 11, de forme cylindrique, possédant une extrémité libre 8 munie d'aspérités, et est implanté dans la partie pleine 4 du bouton poussoir 3 de sorte qu'il se retrouve dans la partie cylindrique creuse 5 dudit bouton 3, dans une position centrale pour laquelle leurs deux axes sont confondus.

Le bouton poussoir 3 enserre le corps cylindrique creux 6 de façon à ce que leurs zones respectives présentant des aspérités annulaires se correspondent en s'imbriquant l'une dans l'autre et qu'un espace libre 9 subsiste entre la partie pleine 4 du bouton poussoir 3 et l'extrémité du corps cylindrique creux 6 lui faisant face.

L'emboîtement de ces deux zones permet de créer une zone de rugosité 10 constituée de points durs, de manière à accroître les forces de frottement en cas de coulissement du bouton poussoir 3 le long du corps cylindrique creux 6. Ces aspérités annulaires constituant la zone de rugosité 10 ont la particularité de se déformer préférentiellement suivant l'axe de poussée et d'avoir, par conséquent, un effet anti-retour. La paroi latérale externe de l'embase élargie 7 du corps cylindrique creux 6 se retrouve au contact de la paroi latérale interne du cylindre avant de la partie cylindrique creuse 5 du bouton poussoir 3, et l'extrémité du cylindre avant est au contact sur , l'embase 7 de façon à éviter au bouton poussoir 3 d'être facilement retiré de la seringue 1.

Le rugueux qui est fixé à la partie pleine 4 du bouton poussoir 3 traverse le canal amont du corps cylindrique creux 6. et son extrémité libre 8 munie d'aspérités débouche dans le canal aval dudit corps 6 à une certaine distance de la charge pyrotechnique 2. Un joint 16, placé dans une gorge circulaire du canal amont, vient en appui contre le rugueux 12, de façon à assurer une bonne étanchéité entre le canal aval dans lequel va se produire la combustion et l'espace libre 9 situé entre le bouton poussoir 3 et l'extrémité du corps cylindrique creux 6.

La seringue 1 dispose d'une sûreté sous la forme d'une butée escamotable constituée par un bouchon 13 muni d'une collerette rigide 14 détachable, ayant le même diamètre que le diamètre extérieur du bouton poussoir 3. Le bouton 13 de forme cylindrique vient s'emboîter autour de l'extrémité sensible de la seringue 1 par laquelle est expulsé le principe actif. Le principe actif, ou tout du moins son emplacement dans la seringue, n'est pas représenté sur les figures, mais quelle que soit le configuration retenue, il est situé en aval de la charge pyrotechnique 2, soit dans le chemin des gaz, soit sur le côté.

La collerette 14 qui est de forme cylindrique est solidaire du bouchon 13, et est bloquée entre ledit bouchon 13 et l'extrémité libre de la partie cylindrique creuse 5 du bouton poussoir 3.

Une zone de prédécoupage, sous la forme d'une rainure circulaire, est réalisée entre la collerette 14 et le bouchon 13 et une tirette 15 fixée à ladite collerette 14 peut être facilement saisie par l'utilisateur pour contribuer à détacher la collerette 14.

Le fonctionnement de cette variante préférée de seringue selon l'invention fait intervenir les étapes suivantes.

L'utilisateur se saisit de la tirette 15 et agit de façon à provoquer la séparation de la collerette 14 et du bouchon 13 suivant la ligne circulaire de prédécoupage. Une fois la collerette 14 enlevée, le bouchon 13 de protection est à son tour retiré et la seringue 1 se retrouve ainsi déverrouillée.

La partie aval de la seringue 1 est mise au contact de la peau du patient à traiter. L'utilisateur exerce alors une pression manuelle sur le bouton poussoir 3 au niveau de sa partie pleine 4, de façon à l'enfoncer. Pour ce faire, il doit fournir un effort pour vaincre les forces de frottement induites par la zone de rugosité 10. Lorsque le bouton poussoir 3 commence à coulisser le long du corps cylindrique creux 6, il entraîne un déplacement linéaire du rugueux 12 dans le corps cylindrique creux 6 jusqu'à ce que son extrémité libre 8 munie d'aspérités atteigne la charge pyrotechnique 2 au niveau de sa partie constituée par la composition à base de Zirconium sensible à la friction. Cette extrémité 8, qui joue le rôle d'un frottoir, induit un frottement au sein de ladite charge 2 qui réagit en s'enflammant. De cette manière, les gaz générés par cette combustion vont provoquer l'initiation de la composition génératrice de gaz. Les gaz résultant de cette deuxième combustion vont permettre d'éjecter le principe actif à travers la peau du patient, soit en déplaçant, par exemple, un piston pour éjecter du principe actif sous forme liquide à travers une buse, soit en créant, par exemple, une onde de choc pour accélérer des particules de principe actif sous forme solide. Le déplacement maximum du bouton poussoir 3 correspond à la venue en butée de l'épaulement interne de la partie cylindrique creuse 5 contre l'embase élargie 7 du corps cylindrique creux 6. Les aspérités annulaires de la zone de rugosité 10 étant aptes à se déformer suivant l'axe de poussée, ont un effet anti-retour pour permettre notamment au bouton poussoir 3 de rester enfoncé, en conservant sa position par rapport au corps cylindrique creux 6. Le joint circulaire 16 permet également d'éviter une remontée intempestive des gaz vers le bouton poussoir 3.

## Revendications

1. Seringue sans aiguille munie d'un dispositif d'initiation comprenant un dispositif de déclenchement et une charge pyrotechnique (2) **caractérisée en ce que** le dispositif de déclenchement comprend un organe de déclenchement (3) relié à un moyen de friction (12) de la charge pyrotechnique (2).

2. Seringue sans aiguille selon la revendication 1 **caractérisée en ce que** le moyen de friction (12) est un rugueux.

3. Seringue sans aiguille selon la revendication 2 **caractérisée en ce que** le rugueux est constitué par une pièce allongée (11) dont une extrémité (8) présente des aspérités.

4. Seringue sans aiguille selon la revendication 3 **caractérisée en ce que** la pièce (11) est rigide et a une forme sensiblement cylindrique.

5. Seringue sans aiguille selon la revendication 3 **caractérisée en ce que** la pièce (11) est solidaire de l'organe de déclenchement (3) de sorte que son déplacement est dicté par le mouvement imprimé audit organe (3).

6. seringue sans aiguille selon la revendication 5 **caractérisée en ce que** l'organe de déclenchement (3) est un bouton poussoir destiné à être actionné manuellement et pouvant coulisser le long d'un corps (6) central creux allongé dans lequel est logée la charge pyrotechnique (2).

7. Seringue sans aiguille selon la revendication 6 **caractérisée en ce que** le bouton poussoir (3) est placé à l'une des extrémités du corps central (6).

8. Seringue sans aiguille selon la revendication 6 **caractérisée en ce que** le rugueux (12) et la charge pyrotechnique (2) sont alignés suivant l'axe du corps creux (6).

9. Seringue sans aiguille selon la revendication 6 **caractérisée en ce que** une zone de rugosité (10) située entre le bouton poussoir (3) et le corps central (6) permet d'accroître les forces de frottement entre ces deux éléments en cas de coulissement de l'un sur l'autre.

10. Seringue sans aiguille selon la revendication 6 **caractérisée en ce que** le bouton poussoir (3) dispose d'une sûreté sous la forme d'une butée escamotable (13, 14) empêchant tout mouvement dudit bouton (3).

11. Seringue sans aiguille selon la revendication 10 **caractérisée en ce que** la butée escamotable, est constituée par un bouchon (13) d'une collerette détachable (14).

12. Seringue sans aiguille selon la revendication 2 **caractérisée en ce que** le rugueux (12) est en acier inoxydable.

## Patentansprüche

1. Nadellose Spritze, die mit einer Zündvorrichtung ausgestattet ist, die eine Auslösevorrichtung und eine pyrotechnische Ladung (2) aufweist, **dadurch gekennzeichnet, dass** die Auslösevorrichtung ein Auslöseorgan (3) aufweist, das mit einem Mittel (12) zum Reiben der pyrotechnischen Ladung (2) verbunden ist.

2. Nadellose Spritze nach Anspruch 1, **dadurch gekennzeichnet, dass** das Reibmittel (12) ein rauflächiges Element ist.

3. Nadellose Spritze nach Anspruch 2, **dadurch gekennzeichnet, dass** das rauflächige Element aus einem länglichen Teil (11) besteht, von dem ein Ende (8) Unebenheiten aufweist.

4. Nadellose Spritze nach Anspruch 3, **dadurch gekennzeichnet, dass** das Teil (11) steif ist und eine im wesentlichen zylindrische Form hat.

5. Nadellose Spritze nach Anspruch 3, **dadurch gekennzeichnet, dass** das Teil (11) fest mit dem Auslöseorgan (3) verbunden ist, so dass seine Verschiebung durch die dem Organ (3) verliehene Bewegung festgelegt wird.

6. Nadellose Spritze nach Anspruch 5, **dadurch gekennzeichnet, dass** das Auslöseorgan (3) ein Druckknopf ist, der von Hand zu betätigen ist und entlang eines mittigen länglichen Hohlkörpers (6) gleiten kann, in dem die pyrotechnische Ladung (2) untergebracht ist.

7. Nadellose Spritze nach Anspruch 6, **dadurch gekennzeichnet, dass** der Druckknopf (3) an einem der Enden des mittigen Körpers (6) angeordnet ist.

8. Nadellose Spritze nach Anspruch 6, **dadurch gekennzeichnet, dass** das rauflächige Element (12) und die pyrotechnische Ladung (2) entlang der Achse des Hohlkörpers (6) fluchtend ausgerichtet sind.

9. Nadellose Spritze nach Anspruch 6, **dadurch gekennzeichnet, dass** eine zwischen dem Druckknopf (3) und dem mittigen Körper (6) angeordnete Rauigkeitszone (10) es ermöglicht, die Reibkräfte zwischen diesen beiden Teilen zu erhöhen, wenn das eine Teil über das andere Teil gleitet.

10. Nadellose Spritze nach Anspruch 6, **dadurch gekennzeichnet, dass** der Druckknopf (3) über eine Sicherung in Form eines entfernbaren Anschlags (13, 14) verfügt, der jede Bewegung dieses Knopfes (3) verhindert.

11. Nadellose Spritze nach Anspruch 10, **dadurch gekennzeichnet, dass** der entfernbare Anschlag aus einem Deckel (13) besteht, der mit einem abnehmbaren Kragen (14) ausgestattet ist.

12. Nadellose Spritze nach Anspruch 2, **dadurch gekennzeichnet, dass** das rauflächige Element (12) aus nichtrostendem Stahl besteht.

## Claims

1. Needle-free syringe provided with an initiation device comprising a triggering device and a pyrotechnic charge (2), **characterised in that** the triggering device comprises a triggering unit (3) which is connected to a friction means (12) for the pyrotechnic charge (2)

2. Needle-free syringe according to claim 1,
**characterised in that** the friction means (12) is a striker.

3. Needle-free syringe according to claim 2,
**characterised in that** the striker consists of an elongate part (11), one end (8) of which has rough parts.

4. Needle-free syringe according to claim 3,
**characterised in that** the part (11) is rigid and has a substantially cylindrical shape.

5. Needle-free syringe according to claim 3,
**characterised in that** the part (11) is integral with the triggering unit (3), such that its displacement is governed by the movement imparted to the said unit (3).

6. Needle-free syringe according to claim 5,
**characterised in that** the triggering unit (3) is a push-button which is designed to be activated manually and can slide along an elongate hollow central body (6) in which the pyrotechnic charge (2) is accommodated.

7. Needle-free syringe according to claim 6,
**characterised in that** the push-button (3) is placed at one of the ends of the central body (6).

8. Needle-free syringe according to claim 6,
**characterised in that** the striker (12) and the pyrotechnic charge (2) are aligned according to the axis of the hollow body (6).

9. Needle-free syringe according to claim 6,
**characterised in that** an area of roughness (10) situated between the push-button (3) and the central body (6) makes it possible to increase the friction forces between these two elements when one of them slides on the other.

10. Needle-free syringe according to claim 6,
**characterised in that** the push-button (3) has a safety device in the form of a retractable stop (13,14) which prevents any movement of the said button (3).

11. Needle-free syringe according to claim 10,
**characterised in that** the retractable stop consists of a stopper (13) which is provided with a detachable collar (14).

12. Needle-free syringe according to claim 2,
**characterised in that** the striker (12) is made of stainless steel.
